(19) Europäisches Patentamt
European Patent Office
Office européen des brevets

(11) **EP 3 289 334 B1**

(12) **FASCICULE DE BREVET EUROPEEN**

(45) Date de publication et mention
de la délivrance du brevet:
**05.03.2025 Bulletin 2025/10**

(51) Classification Internationale des Brevets (IPC):
***G01N 21/25*** *(2006.01)*   ***G01N 21/27*** *(2006.01)*
***G01N 21/64*** *(2006.01)*

(21) Numéro de dépôt: **16724687.5**

(22) Date de dépôt: **28.04.2016**

(52) Classification Coopérative des Brevets (CPC):
**G01N 21/253; G01N 21/274; G01N 21/6428;**
**G01N 21/645;** G01N 2021/6417; G01N 2021/6419;
G01N 2021/6421; G01N 2021/6441;
G01N 2021/6463

(86) Numéro de dépôt international:
**PCT/FR2016/050996**

(87) Numéro de publication internationale:
**WO 2016/174356 (03.11.2016 Gazette 2016/44)**

(54) **MACHINE ET PROCÉDÉ POUR LA DÉTECTION AUTOMATISÉE IN VITRO D'ANALYTES METTANT EN OEUVRE UNE DÉCOMPOSITION SPECTRALE CHROMATIQUE D'UNE RÉPONSE OPTIQUE**

VORRICHTUNG UND VERFAHREN ZUM AUTOMATISCHEN IN-VITRO-NACHWEIS VON ANALYTEN ANHAND EINER SPEKTRALEN ZERLEGUNG EINER OPTISCHEN ANTWORT

DEVICE AND METHOD FOR IN VITRO AUTOMATIC DETECTION OF ANALYTES BASED ON A SPECTRAL DECOMPOSITION OF AN OPTICAL RESPONSE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.04.2015 FR 1553912**

(43) Date de publication de la demande:
**07.03.2018 Bulletin 2018/10**

(73) Titulaire: **bioMérieux**
**69280 Marcy l'Etoile (FR)**

(72) Inventeurs:
• **ROSSI, Veronica Lucia**
**50139 Florence (IT)**
• **FERORELLI, Giuseppe**
**50133 Florence (IT)**
• **SANESI, Antonio**
**50126 Florence (IT)**
• **UBALDINI, Giuseppe**
**50012 Bagno A Ripoli (IT)**

(74) Mandataire: **bioMérieux PI Groupement mandataires**
**bioMérieux**
**69280 Marcy l'Etoile (FR)**

(56) Documents cités:
| | |
|---|---|
| EP-A1- 2 853 884 | EP-A1- 2 902 772 |
| EP-A2- 0 241 268 | WO-A1-2010/009543 |
| WO-A1-2014/045481 | US-A1- 2003 223 059 |
| US-A1- 2007 086 006 | US-B1- 8 868 156 |

**Description**

La présente invention se rapporte à une machine et à des procédés de détection et/ou de quantification in vitro par analyse optique, notamment par fluorimétrie et/ou colorimétrie, d'au moins un analyte présent dans un échantillon, notamment un échantillon biologique.

La présente invention trouve une application dans les instruments automatisés d'analyse in vitro dans le domaine clinique ou dans le domaine industriel.

Dans le domaine clinique, l'analyse peut concerner un prélèvement biologique humain (urine, sang, salive, pus, liquide céphalo- rachidien, etc.), pour la détection ou la quantification d'analytes issus ou non d'un microorganisme externe (bactérie, virus, parasite, anticorps, etc.), notamment sous la forme de tests immunologiques ou de tests de biologie moléculaire.

Dans le domaine industriel, l'analyse peut concerner un échantillon de produit alimentaire, pharmaceutique ou cosmétique, par exemple pour contrôler la qualité microbiologique du produit sous la forme de tests microbiologiques. De tels tests microbiologiques vérifient généralement soit la stérilité (aucun microorganisme ne doit être présent), soit l'absence de bactéries pathogènes (à l'origine d'une infection), soit encore qu'une bactérie commensale (normalement présente chez l'homme et banale en faible concentration) n'est présente qu'en dessous d'un certain seuil.

L'invention peut également trouver une application dans le domaine des analyses dynamiques, c'est-à-dire en temps réel, en l'occurrence dans les tests immunologiques et les tests de biologie moléculaire où les réactions immunologiques/biologiques peuvent être pilotées ou surveillées dans le temps.

De tels systèmes ou procédés de détection et/ou de quantification in vitro sont connus, en particulier dans les instruments automatisés de diagnostic in vitro par fluorimétrie, comme par exemple dans les documents US2003/223059 A1, EP 0 864 089 B1, EP 0 871 863 B1, EP 0 241 268 A1, US 5 757 013 A, EP 0 802 413, et WO 2004/055502 A2, qui emploient des sources de rayonnement du type source de lumière pulsé, lampe laser ou lampe à arc.

Le demandeur commercialise par ailleurs des machines selon ces principes sous la dénomination commerciale VIDAS®, notamment la machine VIDAS® 3, ou la machine VIDAS® Legacy BLUE. Ces machines permettent notamment une automatisation du processus d'analyse.

Dans les machines et procédés selon l'art antérieur, on trouve donc :

- une ou plusieurs zones de réception destinées à recevoir chacune un échantillon à analyser ;
- des moyens de préparation et de réaction automatisés qui préparent l'échantillon et le font réagir avec des réactifs pour fournir une solution de réaction ;
- plusieurs zones d'analyse optique destinées à recueillir chacune une solution de réaction obtenue par réaction avec un échantillon.
- un dispositif de lecture optique capable de détecter et/ou quantifier la réponse optique de la solution de réaction à une stimulation électromagnétique, le dispositif de lecture optique comportant au moins une source de rayonnement électromagnétique capable d'illuminer une zone d'analyse et un récepteur photoélectrique agencé pour recueillir un rayonnement optique provenant de la solution de réaction contenue dans la zone d'analyse et capable de détecter la réponse optique de la solution de réaction.

Dans les machines selon l'art antérieur, il est connu que le dispositif de lecture optique soit porté par un chariot mobile de la machine qui est motorisé et dont les déplacements sont commandés automatiquement pour amener le dispositif de lecture optique dans différentes positions correspondant chacune à une zone d'analyse. Ainsi, une telle machine permet, avec un même dispositif de lecture mobile, de venir analyser la réponse lumineuse dans plusieurs zones d'analyse distinctes, successivement, ceci de manière automatisée. On notera donc que, dans ces machines, c'est bien le dispositif de lecture qui est mobile pour assurer la lecture optique des différentes zones d'analyse, et non pas les zones qui seraient déplacées tour à tour devant un dispositif de lecteur fixe. Cela nécessite donc de concevoir des dispositifs de lecture optique simples, puisqu'ils doivent être mobiles.

Dans l'état de la technique, les machines mettent en œuvre un procédé pour la détection et/ou la quantification automatisée in vitro d'analytes contenus dans les échantillons, qui comporte généralement les étapes suivantes :

- l'échantillon est mis en situation de réagir avec un ou plusieurs réactifs pour produire directement ou indirectement une solution de réaction comportant, en présence d'un analyte déterminé dans l'échantillon, un agent ayant des propriétés optiques prédéterminées, notamment des propriétés de fluorescence ;
- la solution de réaction est illuminée avec un rayonnement électromagnétique ;
- la réponse optique de la solution de réaction est détectée;
- de la réponse optique est déduite la présence et/ou la quantification de l'analyte.

De manière classique, l'analyse des signaux en provenance du ou des moyens photo-détecteurs s'effectue par un traitement analogique ou numérique, avec notamment un algorithme de détection d'une intensité lumineuse à une fréquence/longueur d'onde prédéterminée qui est caractéristique de l'agent représentatif de la présence/quantité d'analyte à détecter.

Pour cela, on utilise généralement un capteur photoélectrique et la réponse optique de la solution de réaction est filtrée par un filtre optique agencé entre la zone d'analyse et le capteur photoélectrique ne laissant passer la lumière que pour une longueur d'onde ou une bande de longueurs d'onde. De la sorte, le capteur photoélectrique délivre un signal représentatif de l'intensité de la réponse optique à cette longueur d'onde ou une bande de longueurs d'onde. Le filtrage de la réponse optique de la solution de réaction est une étape essentielle des procédés mis en œuvre dans les machines connues car elle permet de distinguer la partie de réponse qui est liée à l'agent optique (en l'occurrence un agent fluorescent) et qui permet de quantifier la présence de l'agent, d'une éventuelle partie de réponse optique qui pourrait être la simple transmission du rayonnement d'excitation et/ou qui pourrait provenir de sources de rayonnements optiques parasites.

Les machines et procédés de l'art antérieur sont très satisfaisants sur de nombreux points. Cependant, il est apparu le besoin de pouvoir améliorer encore la précision de mesure de ces machines, pour affiner les résultats. En parallèle il est apparu le besoin de pouvoir encore augmenter la capacité des machines, c'est-à-dire leur capacité à délivrer des résultats d'analyses plus nombreux, y compris sur un même échantillon, sans toutefois augmenter de manière significative l'encombrement des machines.

Dans ce but, l'invention propose une machine pour la détection et/ou la quantification automatisée in vitro d'analytes contenus dans des échantillons telle que définie par la revendication 3.

L'invention concerne aussi un procédé pour la détection et/ou la quantification automatisée in vitro d'analytes contenus dans des échantillons, tel que défini par la revendication 1.

Des modes de réalisation de l'invention sont définis par les revendications dépendantes.

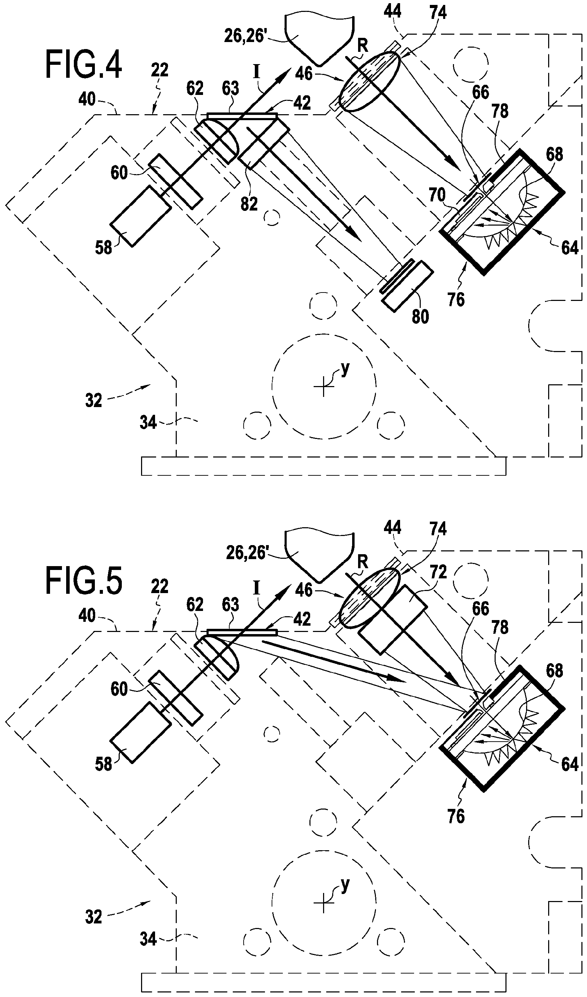

Diverses autres caractéristiques ressortent de la description faite ci-dessous en référence aux dessins annexés qui, à l'exception de la figure 4, montrent, à titre d'exemples non limitatifs, des formes de réalisation de l'objet de l'invention.

- La **figure 1** est une vue schématique en perspective d'une machine de détection et/ou de quantification in vitro par analyse optique, notamment par fluorimétrie et/ou colorimétrie, dans laquelle l'invention peut être intégrée, ladite machine intégrant plusieurs barrettes comportant chacune une zone d'analyse.
- La **figure 2** est une vue illustrant une barrette comportant une zone d'analyse ;
- la **figure 3** est une vue schématique en perspective illustrant un exemple de réalisation d'un chariot mobile portant un dispositif de lecture optique pour lire la réponse optique d'une solution de réaction contenue dans une zone d'analyse ;
- la **figure 4** illustre un premier mode de réalisation d'un dispositif de lecture optique susceptible d'être monté sur un chariot mobile et comportant un spectromètre et non conforme à l'invention;
- la **figure 5** est une vue similaire à celle de la **figure 4** illustrant un second mode de réalisation d'un dispositif de lecture optique utilisé dans l'invention ;
- la **figure 6** illustre une décomposition spectrale chromatique obtenue dans le cadre de l'invention ;
- La **figure 7** illustre la superposition de trois décompositions spectrales chromatiques obtenues séparément, par un procédé et un dispositif de l'invention, pour trois agents optiques séparés ;
- La **figure 8** illustre la décomposition spectrale chromatique obtenue, par un procédé et un dispositif de l'invention, pour une solution comprenant simultanément les trois agents optiques ;
- La **figure 9** illustre, en superposition avec la décomposition spectrale chromatique de la **figure 8,** une courbe S globale de modélisation de la décomposition spectrale de la **figure 8** ; la **figure 10** illustre, en superposition avec la décomposition spectrale chromatique de la **figure 8,** trois courbes composantes G525, G605, G705 de la courbe S globale de modélisation.

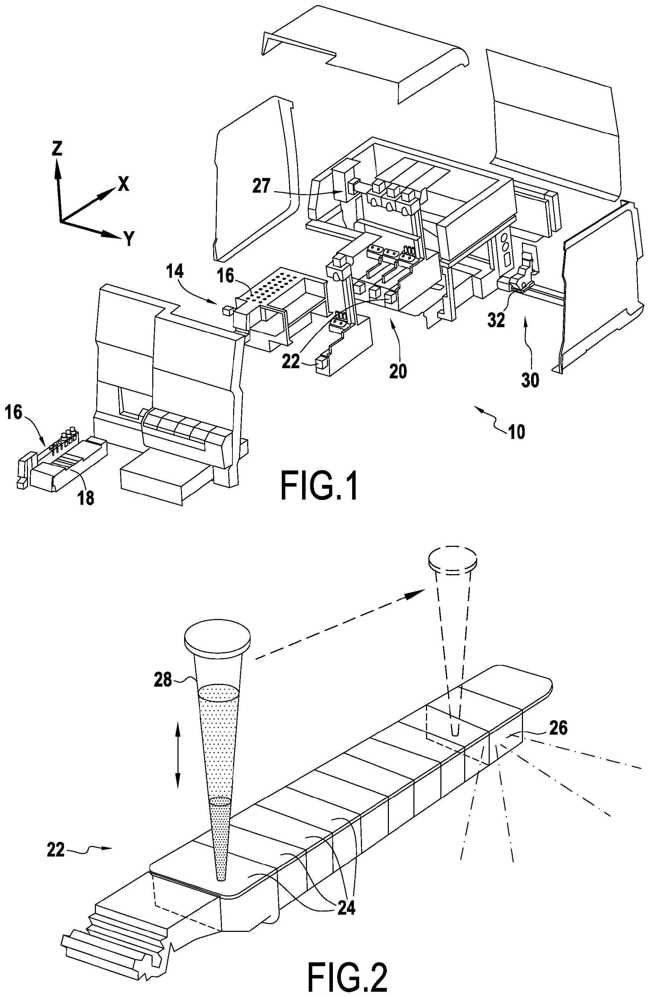

On a illustré sur la **figure 1** une machine pour la détection et/ou la quantification automatisée in vitro d'analytes contenus dans des échantillons.

Plus précisément, on a illustré le cas d'une machine automatisée assurant non seulement les étapes de détection et/ou de quantification mais aussi une ou plusieurs étapes de préparation d'une solution de réaction à partir d'un échantillon.

La machine ici décrite correspond notamment à une machine du type commercialisé par la demanderesse sous la dénomination commerciale «VIDAS® 3 ». Ce type de machine permet de réaliser des analyses biologiques sur des échantillons biologiques de manière automatisée, et avec la possibilité de pratiquer, sur une même machine, différentes analyses sur un même échantillon ou sur des échantillons différents.

Une machine **10** de ce type est illustrée en vue schématique en perspective éclatée sur la **figure 1.** Dans une partie inférieure gauche, appelée partie pré-analytique **14** de la machine, on peut distinguer une série de trois tiroirs primaires **16** et un tiroir secondaire **18,** qui sont agencés côte-à-côte. Chacun de ces tiroirs **16, 18** est prévu pour être mobile, indépendamment les uns des autres, entre une position de chargement et une position d'utilisation, ici par un coulissement linéaire selon une trajectoire horizontale perpendiculaire à une face avant de la machine, selon une direction **X** qualifiée de longitudinale. Les notions de « avant », « arrière », « horizontal », « vertical » etc... sont utilisées ici à titre indicatif en référence à l'orientation normale d'une telle machine telle qu'illustrée sur les figures. Les tiroirs primaires **16** sont prévus pour recevoir par exemple des échantillons, des produits diluants, des réactifs, des produits de référence etc... contenus par exemple dans des récipients de formes variées. Chaque tiroir primaire **16** peut ainsi porter plusieurs récipients, chaque récipient étant de préférence reçu dans un emplacement prédéterminé du tiroir primaire **16**. Chacun des tiroirs primaires **16** peut être amovible de la machine pour permettre une préparation et un chargement des tiroirs en dehors de la machine, permettant ainsi de faire cette étape de préparation pendant que la machine fonctionne avec d'autres tiroirs primaires en position d'utilisation. Chaque tiroir primaire **16** peut comporter, à son extrémité avant, une poignée de préhension. Le tiroir secondaire **18,** dans cet exemple d'application, est par exemple destiné à recevoir des outils d'analyse, par exemple des outils jetables utilisés par la machine dans le processus d'analyse automatisée. Ces outils, par exemple des embouts de pipette ou des coupelles de dilution, peuvent être portés par des plateaux amovibles que l'on peut déposer, de préférence à des endroits prédéterminés, sur le tiroir secondaire **18**. Le tiroir secondaire **18** peut lui aussi être amovible de manière à ce que plusieurs tiroirs secondaires **18** puissent être utilisés alternativement sur la machine.

Par ailleurs, on distingue sur la **figure 1** une partie droite de la machine, parfois qualifié de partie analytique **20,** et qui comporte elle aussi des emplacements pouvant par exemple recevoir des barrettes d'analyse, qui peuvent être jetables, telles que celles illustrées à la **Figure 2.** La barrette d'analyse **22** est une pièce généralement réalisée en matière plastique, notamment dans le cas où, comme dans cet exemple, elle est jetable. La barrette **22** comporte un corps principal délimitant une série de réceptacles **24** ouverts vers le haut. La barrette est allongée selon une direction longitudinale de telle sorte que les réceptacles **24** sont alignés selon une rangée unique selon cette direction longitudinale qui est destinée à correspondre à la direction avant-arrière de la machine **10.** Avant l'utilisation de la barrette **22,** au moins certains des réceptacles **24,** voire de préférence tous les réceptacles, sont refermés par un film étanche qui recouvre de manière hermétique la face supérieure de barrette **22,** de sorte que les réceptacles **24** sont isolés les uns des autres. Avant l'utilisation de la barrette, les réceptacles **24** sont soit vides, soit remplis avec un produit qui peut par exemple comprendre une solution tampon, une solution de lavage, un diluant, un réactif, etc... Toutefois, dans l'exemple illustré, le dernier réceptacle ou réceptacle arrière **26** de la barrette est destiné à coïncider géographiquement, lorsque la barrette est en place dans la machine dans une position d'utilisation, avec une zone d'analyse optique **26'** de la machine.

Dans l'exemple illustré, une barrette est adaptée à un ou plusieurs types d'analyse par la nature des produits qui sont prévus dans les différents réceptacles, dont tous ne seront pas nécessairement utilisés pour une analyse donnée.

Dans la machine illustrée, la machine **10** est prévue pour recevoir simultanément plusieurs barrettes d'analyse **22.** Ces barrettes **22** sont prévues pour être disposées parallèlement l'une à l'autre, côte à côte, les barrettes étant ainsi alignées selon une direction transversale **Y** perpendiculaire à leur direction longitudinale **X.** Dans l'exemple illustré, la machine **10** est prévue pour pouvoir recevoir ainsi 12 barrettes d'analyse **22** côte-à-côte en position d'utilisation. Dans cette position, le réceptacle arrière **26** de chaque barrette **22** coïncide avec une zone d'analyse optique correspondante **26'** de la machine. En d'autres termes, la machine comporte plusieurs zones d'analyse **26'** et, dans le cas d'une machine utilisant des barrettes d'analyse telles que les barrettes **22,** chaque zone d'analyse **26'** de la machine est prévue pour recevoir le réceptacle arrière **26** d'une barrette. Dans la mesure où ce réceptacle arrière **26** contient une solution finale de réaction, cette solution finale de réaction est recueillie dans la zone d'analyse **26'** correspondante.

Le fonctionnement de la machine **10** illustrée va être décrit de manière succincte. Un opérateur charge, dans les récipients des tiroirs primaires **16,** un ou plusieurs échantillons à analyser. Il peut aussi charger, dans ces récipients, des produits nécessaires à la préparation de l'échantillon et/ou à la préparation de la réaction. Dans le tiroir secondaire **18,** il aura procédé au chargement des produits nécessaires à la préparation.

Dans l'exemple illustré, la machine **10** comporte des moyens de préparation et de réaction automatisés qui préparent l'échantillon et le font réagir avec des réactifs pour fournir une solution finale de réaction dont la réponse optique à un rayonnement électromagnétique d'excitation permettra de déduire des résultats d'analyse.

La machine **10** comporte pour ce faire un dispositif de pipetage mobile **27** qui est de préférence mobile dans les trois directions : longitudinale **X** (avant/arrière), transversale **Y** (gauche/droite) et verticale **Z** (haut/bas). Ce dispositif de pipetage mobile **27** est capable de prélever un embout de pipette individuel **28** (visible sur **Figure 2)** dans le tiroir secondaire **18** et, à l'aide de cet embout de pipette individuel **28,** de prélever ou déposer des produits (échantillons, réactifs, diluants, solutions tampon, etc...). Les produits peuvent être prélevés depuis les récipients des tiroirs primaires ou depuis les réceptacles d'une barrette d'analyse. Les produits et échantillons prélevés peuvent être mélangés soit dans l'embout de pipette individuelle, soit dans des coupelles de dilution présentes dans le tiroir secondaire **18,** soit dans l'un des réceptacles d'une des barrettes d'analyse **22.** Pendant ces différentes étapes, l'analyte présent dans l'échantillon est amené à réagir avec les produits, notamment avec les réactifs, avec lesquels il est mis en contact. Parmi les produits mis en œuvre figure un précurseur d'un agent optique, cet agent optique ayant au moins une propriété optique prédéterminée (couleur, fluorescence, phosphorescence,...).

En fonction de la présence de l'analyte recherché, et en fonction éventuellement de sa concentration, le précurseur va causer la formation d'une quantité de cet agent optique dans une solution finale de réaction. Cette solution finale de réaction possède donc un agent optique, par exemple fluorescent, en une concentration qui témoigne de la présence de l'analyte recherché dans l'échantillon initial. De manière connue, on cherche donc à déterminer la réponse optique de cette solution finale de réaction, et notamment à détecter la propriété optique prédéterminée de l'agent optique, pour en déduire un résultat d'analyse.

De préférence, l'agent optique n'est présent dans la solution de réaction ou ne possède la propriété optique prédéterminée qu'en présence de l'analyte déterminé. De préférence, l'intensité de la propriété optique prédéterminée est une fonction de la quantité ou concentration de l'analyte dans l'échantillon déterminé. Avantageusement, cette propriété optique prédéterminée est détectable par analyse d'une décomposition spectrale chromatique d'un rayonnement émis par la solution de réaction lorsque ce rayonnement est soumis à un rayonnement électromagnétique incident.

L'agent optique est par exemple un agent fluorescent et la propriété optique prédéterminée est l'émission fluorescente, qui peut par exemple être caractérisée par une décomposition spectrale chromatique particulière lorsque cette solution de réaction est illuminée par un rayonnement électromagnétique d'excitation adapté.

L'agent optique pourrait aussi être un agent coloré auquel cas la propriété optique prédéterminée est la couleur, qui peut être déterminée par un spectre d'absorption chromatique lorsque cette solution de réaction est illuminée par un rayonnement électromagnétique incident adapté. On verra toutefois dans ce cas que le système devrait fonctionner en transmission avec un alignement entre la source de rayonnement électromagnétique (ou une réflexion de celle-ci) et le récepteur.

Pour ce faire, il est connu de disposer une certaine quantité de la solution finale de réaction dans une zone d'analyse optique **26'**, qui dans l'exemple illustré, coïncide avec le réceptacle arrière **26** de la barrette d'analyse **22** en position d'utilisation. Il est également connu, notamment de la machine VIDAS® 3 mentionnée plus haut, qu'un dispositif de lecture optique **30** soit utilisé, comportant au moins une source de rayonnement électromagnétique capable d'illuminer la zone d'analyse et un récepteur photoélectrique agencé pour recueillir un rayonnement optique provenant de la solution de réaction contenue dans la zone d'analyse. Le dispositif de lecture peut ainsi détecter la réponse optique de la solution finale de réaction et, en fonction de cette lecture optique, il devient possible de déterminer la présence, voire la concentration, de l'agent optique spécifique de l'analyse.

On comprend donc du fonctionnement décrit ci-dessus que la machine **10** est capable d'avoir plusieurs barrettes d'analyse **22** simultanément engagées dans la machine **10** et donc capable de procéder en parallèle à autant d'analyses différentes, chacune étant susceptible de produire une solution finale de réaction dont il faut être capable de détecter la réponse optique. On notera que deux barrettes d'analyse distinctes peuvent être utilisées et engagées en parallèle dans la machine pour faire deux analyses distinctes d'un même échantillon, pour faire la même analyse sur des échantillons différents chargés dans un tiroir primaire **16**, pour faire la même analyse sur deux parties d'un même échantillon, par exemple à titre de redondance, etc... . Dans ce cas, les deux réceptacles arrière **26** des deux barrettes sont destinés à coïncider avec autant de zones d'analyse optique **26'** considérées alors comme recueillant chacune une solution finale de réaction à analyser.

Pour ce faire, le dispositif de lecture optique **30** est monté sur un chariot mobile **32**. Ce chariot mobile **32** est motorisé et ses déplacements sont commandés automatiquement par la machine **10** pour amener le dispositif de lecture **30** dans différentes positions. Au moins certaines de ces positions correspondent à une zone d'analyse. Dans l'exemple illustré, le chariot mobile **32** est mobile uniquement selon une direction, en l'occurrence la direction transversale **Y** de la machine **10** pour pouvoir être amené dans des positions, selon la direction transversale, correspondant à la position transversale de différentes zones d'analyse **26'** coïncidant avec les réceptacles arrière des barrettes d'analyse **22** susceptibles d'être engagées dans la machine **10**. De préférence, le chariot mobile **32** est susceptible d'être amené dans autant de positions différentes qu'il y a de zones d'analyse **26'** différentes dans la machine.

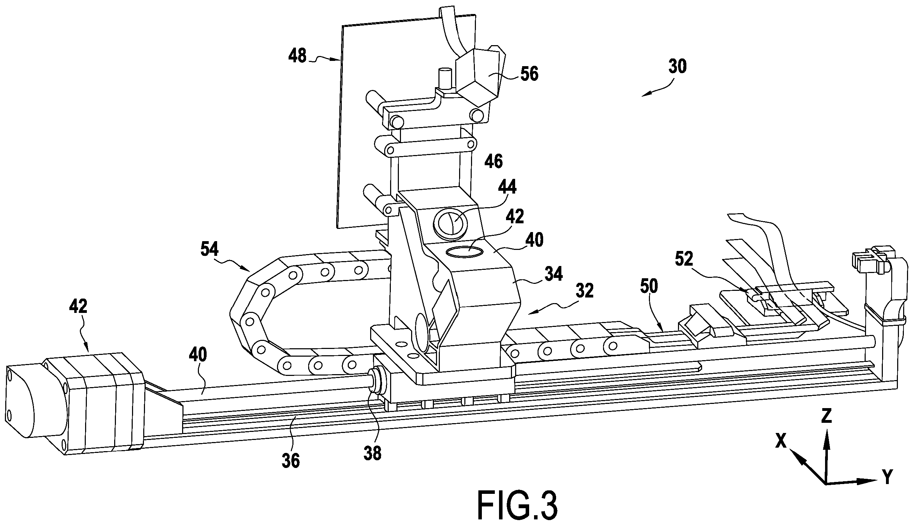

On a illustré sur la **figure 3** un exemple de réalisation possible pour des moyens de commande assurant le déplacement du chariot mobile **32**. Le chariot mobile **32** comporte un châssis **34** qui, à son extrémité inférieure, coopère avec une glissière **36** qui assure le guidage du chariot mobile **32** selon la direction transversale **Y.** Par ailleurs, l'extrémité inférieure du châssis **34** est pourvue d'un système d'écrou **38** qui coopère avec une vis d'entraînement **40** s'étendant selon un axe transversal. La vis d'entraînement **40** est pilotée en rotation par un moteur électrique **42**. On comprend donc que le pilotage adéquat du moteur électrique **42** dans un sens de rotation ou dans l'autre va provoquer, par l'intermédiaire du système vis/écrou formé de la vis d'entraînement **40** et de l'écrou **38** lié au chariot **32,** le déplacement du chariot **32** selon la direction transversale, dans un sens ou dans un autre. Par ailleurs, un système de détection de position du chariot, qui peut reposer par exemple sur les informations délivrées par un encodeur rotatif associé au moteur **42** ou sur les informations délivrées par des capteurs agencés sur la trajectoire du chariot mobile **32,** peut avantageusement être prévu pour connaître la position du chariot mobile **32** le long de la glissière **36**. La glissière **36** est fixe dans la machine, et le moteur **42** ainsi que la vis **40** sont aussi fixes dans la machine si l'on excepte leur mouvement de rotation sur eux-mêmes. Ainsi, le moteur électrique **42** commande le déplacement du chariot **32**. Le moteur électrique **42** est lui-même commandé par une unité centrale de commande de la machine **10**. Bien entendu, d'autres moyens de déplacement du chariot mobile pourraient être prévus. De même, le chariot mobile pourrait se déplacer sur une trajectoire linéaire comme dans l'exemple de réalisation, ou sur une trajectoire courbe ou encore avoir la capacité d'être déplacé selon deux directions, dans un plan, ou encore selon trois directions dans l'espace. De même, les moyens de commande assurant le déplacement du chariot pourraient comprendre d'autres actionneurs et ou d'autres mécanismes de transformation du mouvement de l'actionneur en un déplacement du chariot.

Dans l'exemple de réalisation illustré, le châssis **34** du chariot mobile comporte une zone de travail munie de deux surfaces inclinées l'une par rapport à l'autre, s'étendant toutes les deux selon une direction transversale. Une première surface de travail **40,** horizontale dans l'exemple de réalisation, est munie d'une première fenêtre **42.** Une seconde surface de travail **44,** contenant la direction transversale mais inclinée par rapport à la direction longitudinale, présente une seconde fenêtre **46.** Ces deux surfaces de travail sont destinées à être amenées en regard d'une zone d'analyse **26'** afin que le dispositif de lecture puisse, au travers d'une des fenêtres, émettre un rayonnement électromagnétique d'excitation, par exemple un rayonnement optique dans le domaine visible, ultraviolet, ou infrarouge, en direction de la zone d'analyse **26',** et de recueillir la réponse optique de la zone d'analyse **26',** au travers de l'autre fenêtre.

On comprend donc que, dans l'exemple illustré sur les figures, le chariot **32** embarque à la fois une source de rayonnement électromagnétique d'excitation et un récepteur photoélectrique. Cependant, en variante, on pourrait prévoir que seul le récepteur photoélectrique soit embarqué sur le chariot mobile **32,** la source de rayonnement électromagnétique d'excitation étant par exemple fixe dans la machine. On voit par ailleurs que, au moins dans le mode de réalisation illustré, le chariot **32** comporte une carte électronique **48** assurant notamment au moins en partie le pilotage et le traitement de signal de la source de rayonnement électromagnétique et du récepteur photoélectrique. Cette carte électronique **48** est mobile avec le chariot **32** et les signaux électriques utiles à la carte **48** sont transmis à une unité centrale de commande de la machine par un faisceau électrique **50** qui comporte de préférence au moins une partie flexible, donc déformable de manière réversible, pour permettre les mouvements du chariot. Ce faisceau **50** est par exemple relié à un bornier fixe de la machine **10.** Dans l'exemple illustré, il est reçu dans un guide déroulant **54** à maillons articulés pour en guider la déformation lors des déplacements du chariot, évitant ainsi que le faisceau ne vienne interférer dans le mécanisme de déplacement du chariot. On note par ailleurs que le chariot mobile **32** peut aussi embarquer d'autres dispositifs tels par exemple qu'un lecteur optique de codes-barres **56.** De préférence, la carte électronique **48** délivre en sortie des signaux électriques numériques. Ainsi, les signaux électriques qui sont transmis dans le faisceau électrique **50** sous forme numérique sont bien moins sensibles que des signaux analogiques aux perturbations qui peuvent être engendrées autour de ce faisceau.

Un premier mode de réalisation d'un dispositif de lecture embarqué sur un chariot mobile **32,** est illustré sur la **figure 4.** Dans cette vue en coupe par un plan perpendiculaire à la direction transversale de déplacement du chariot mobile, on trouve tout d'abord, fixée sur le châssis **34,** une source de rayonnement électromagnétique **58** destinée à exciter l'agent optique présent dans la solution finale de réaction contenue dans la zone d'analyse **26'.** Sur la **figure 4,** on a illustré d'ailleurs la présence d'un réceptacle arrière **26** d'une barrette **22** coïncidant avec une zone d'analyse **26',** dans une position relative de travail par rapport au dispositif de lecture **30** qui permet à ce dernier à la fois d'exciter l'agent optique et d'en détecter la réponse optique.

Sur la **figure 4,** on voit que le dispositif de lecture **30** comporte une source de rayonnement électromagnétique qui, dans ce mode de réalisation est fixée sur le châssis **34** du chariot mobile **32,** en dessous de la première surface de travail **42.** Elle peut être avantageusement orientée de manière à émettre un rayonnement électromagnétique d'excitation en direction de la zone d'analyse **26'** au travers de la première fenêtre **42.** Toutefois, un dispositif de guidage optique tel qu'une fibre optique, un ou plusieurs miroirs, un ou plusieurs prismes, etc... peuvent être utilisés pour guider le rayonnement de la source **58** vers la première fenêtre **42,** auquel cas la source **58** pourrait être disposée autrement sur le chariot mobile, par exemple en étant montée sur la carte électronique **48.** La source de rayonnement électromagnétique peut être une source de rayonnement dans le domaine optique, c'est-à-dire un rayonnement dont la longueur d'onde est comprise entre 10 nm et 1 mm, incluant notamment les rayonnements ultraviolets, la lumière visible et les rayonnements infrarouges. Dans l'exemple illustré, la source **58** est une source monochromatique. Cependant, en variante, on peut envisager une source polychromatique, ayant un spectre chromatique discret, ou continu ou mixte. De même, la source **58** peut être constituée d'un composant unique, par exemple un filament incandescent, une diode électroluminescente, une diode laser, ou un tube fluorescent, ou être constituée de plusieurs composants combinés, de même type ou de types différents. Le rayonnement issu de la source **58** peut éventuellement être conditionné par un système de conditionnement pouvant comporter, comme illustré sur la **figure 4,** un filtre chromatique **60** (par exemple passe-haut, passe-bas, passe-bande, et/ou polarisant, etc...), un diaphragme (non représenté), une ou plusieurs lentilles **62.** Par exemple, un filtre **60** laissant passer les longueurs d'ondes utiles pour l'excitation, permet de limiter la présence d'autres longueurs d'ondes qui pourraient perturber la mesure. Le système de conditionnement est de préférence porté par le châssis **34** et agencé entre la source **58** et la première fenêtre **42.** Dans l'exemple illustré, il est prévu, au niveau de la fenêtre **42,** un séparateur de faisceau, par exemple sous la forme d'une lame à faces parallèles partiellement réfléchissante **63,** ou d'un prisme semi-réfléchissant, qui prélève une partie du rayonnement émis par la source **58** pour un besoin qui sera décrit plus loin. Dans l'exemple illustré, le séparateur de faisceau **63** comprend donc une lame semi-réfléchissante, à faces parallèles, qui est agencée au niveau de la fenêtre **42** entre la source **58** et la zone d'analyse **26'.**

Elle s'étend dans un plan qui forme un angle à 45° par rapport à une direction principale d'incidence **I** du rayonnement électromagnétique émis par la source **58.** Comme illustré sur les **figures 4** et **5,** le rayonnement incident émis par la source **58** illumine la zone d'analyse **26'.** Dans l'exemple illustré on voit que la zone d'analyse **26',** qui coïncide avec le réceptacle arrière **26** d'une barrette d'analyse dans l'exemple illustré, présente au moins une fenêtre d'entrée du rayonnement incident, réalisée en matériaux transparent pour le rayonnement de la source **58.** Dans le cas présent, cette fenêtre est constituée par un pan de paroi latérale de du réceptacle arrière **26** qui s'étend sensiblement perpendiculairement à une direction principale d'incidence **I** du rayonnement électromagnétique émis par la source **58.** Ainsi, la solution de réaction finale contenue dans la zone d'analyse est illuminée par le rayonnement émis par la source **58.**

Dans l'exemple illustré, le chemin optique le plus court entre la source de rayonnement électromagnétique d'excitation **58** et la zone d'analyse **26'** s'étend selon une ligne droite, au sens que le système de conditionnement optique du rayonnement d'excitation émis par la source **58** ne provoque pas de changement de direction de l'axe optique entre ces deux éléments. Ce chemin optique le plus court définit donc une direction principale incidente **I** pour le rayonnement électromagnétique d'excitation émis par la source **58** en direction de la zone d'analyse **26'.** Dans l'exemple illustré, on voit que cette direction principale incidente **I** est inclinée par rapport à la première face de travail **40** du châssis **34** d'un angle qui peut être compris entre 30 et 60 degrés, par exemple 45°, ces valeurs étant simplement illustratives de l'exemple de réalisation. Cette direction principale incidente **I** est sensiblement parallèle à la seconde surface de travail **44** du châssis. Dans l'exemple, la lame partiellement réfléchissante **63** présente une surface plane semi-réfléchissante qui est orientée avec un angle, par exemple à 45°, par rapport à la direction principale incidente **I** pour permettre de réfléchir une partie de l'intensité du faisceau, par exemple 50% ou moins, vers un canal de référence, tel que cela sera décrit plus loin, en laissant l'autre partie de l'intensité du faisceau se diriger vers la zone d'analyse **26'.**

Le chariot mobile **32** comporte un récepteur photoélectrique qui dans, l'exemple illustré, fait partie d'un spectromètre **64.**

Le spectromètre utilisé dans l'exemple de réalisation illustré est un composant opto-électronique capable de délivrer un signal électronique représentatif de la décomposition spectrale chromatique d'un rayonnement reçu par le spectromètre. Pour un rayonnement électromagnétique dans le domaine de l'optique, notamment dans le domaine du visible, de l'infrarouge et/ou de l'ultraviolet, le rayonnement étant considéré comme une superposition d'ondes élémentaires monochromatiques, cette décomposition est une représentation de la distribution de l'intensité de chaque onde élémentaire en fonction de la longueur d'onde de l'onde élémentaire.

De par l'installation du spectromètre **64** sur le chariot mobile **32,** le rayonnement reçu par le spectromètre peut notamment comprendre la réponse optique de la solution finale de réaction contenue dans une zone d'analyse **26',** lorsque cette dernière est illuminée par le rayonnement électromagnétique d'excitation émis par la source **58.**

Dans l'exemple illustré, le spectromètre **64** comporte essentiellement un diaphragme d'entrée **66,** un élément de dispersion chromatique **68** et un capteur photoélectrique **70.**

Le spectromètre **64** présente un champ de vision correspondant à la portion de l'espace vu par le capteur photoélectrique **70** au travers de l'élément de dispersion chromatique **68** et du diaphragme d'entrée **66.**

Dans l'exemple illustré, le spectromètre **64** est agencé sur le châssis **34** de manière que, pour une position déterminée du chariot mobile **32,** son champ de vision, au travers d'un système de conditionnement optique du récepteur, englobe la zone d'analyse **26'** correspondante de manière qu'un rayonnement optique émis par une solution finale de réaction contenue dans la zone d'analyse **26'** soit dirigé par le système de conditionnement optique du récepteur vers le diaphragme d'entrée **66** du spectromètre selon une direction adéquate. Le système de conditionnement optique du récepteur peut être porté par le châssis **34** et être agencé entre le spectromètre et la seconde fenêtre **46.** Le système de conditionnement optique du récepteur peut, de manière optionnelle, comporter notamment un filtre chromatique (non présent dans l'exemple de réalisation de la **figure 4,** mais qui pourrait être par exemple un filtre passe-haut, passe-bas, passe-bande, et/ou polarisant, etc...), un diaphragme (non représenté), une ou plusieurs lentilles **74.** Un dispositif de guidage optique tel qu'une fibre optique, un ou plusieurs miroirs, un ou plusieurs prismes, un guide optique de forme tronconique, tel qu'illustré à la **figure 4,** etc. peuvent être utilisés pour guider le rayonnement depuis la seconde fenêtre **46** vers le spectromètre **64,** de préférence vers le diaphragme d'entrée **66,** selon une direction d'entrée prédéfinie. Par exemple, le système de conditionnement optique du récepteur peut être prévu pour délivrer, à l'entrée du spectromètre, un faisceau collimaté de rayonnement en provenance de la zone d'analyse **26'.**

Le diaphragme d'entrée **66** peut être un diaphragme ponctuel, sensiblement circulaire et perpendiculaire à la direction d'entrée prédéfinie du spectromètre **64** ou, de préférence, un diaphragme linéaire réalisé sous la forme d'une fente.

Le capteur photoélectrique **70** peut être un capteur linéaire ou un capteur bidimensionnel. Il peut s'agir d'un capteur de technologies CCD ou CMOS ou autre.

L'élément de dispersion chromatique **68** provoque une dispersion chromatique d'un faisceau incident, au sens qu'un faisceau incident polychromatique, par exemple un faisceau parallèle, voit, par interaction avec l'élément de dispersion chromatique, ses différentes composantes chromatiques être déviées de leur trajectoire d'un angle qui est dépendant de la longueur d'onde de la composante chromatique considérée. Un élément de dispersion chromatique **68** peut comporter un réseau de diffraction. Un réseau de diffraction peut notamment comporter une série de fentes parallèles (réseau en transmission), ou de rayures réfléchissantes (réseau en réflexion). Ces fentes ou rayures sont espacées de manière régulière, l'espacement étant appelé le « pas » du réseau. Un élément de dispersion chromatique **68** pourrait aussi être réalisé sous la forme d'un ou plusieurs dioptres, par exemple par utilisation d'un prisme de réfraction, dans lequel la dispersion chromatique est obtenue par réfraction, voire sous la forme d'une combinaison d'un ou plusieurs dioptres et d'un ou plusieurs réseaux de diffraction. Dans l'exemple illustré, le réseau de diffraction est ici représenté comme faisant partie d'une surface de réflexion concave renvoyant le rayonnement dispersé vers le capteur photoélectrique, ce qui permet une plus grande compacité du spectromètre.

Dans l'exemple illustré, le chemin optique le plus court entre la zone d'analyse **26'** et le diaphragme d'entrée **68** du spectromètre **64** s'étend selon une ligne droite, au sens que le système de conditionnement optique de la réponse optique ne provoque pas de changement de direction de l'axe optique. Ce chemin optique le plus court définit donc une direction principale retour **R** de la réponse optique depuis la zone d'analyse **26'** vers le spectromètre **64**. Dans l'exemple illustré, on voit que cette direction principale retour **R** est perpendiculaire à la seconde surface de travail **44** du châssis. On notera donc que, de préférence, notamment pour une analyse par fluorimétrie, avec détection d'agent optiques fluorescents, la direction principale d'incidence **I,** prise dans le sens allant de la source **58** vers la zone d'analyse **26',** et la direction principale retour **R,** prise dans le sens allant de la zone d'analyse **26'** vers le spectromètre **64** ne sont pas à la fois parallèles et de même sens. Au contraire, elles forment entre elles un angle minimal par exemple d'au moins 45°, avec la convention que des directions parallèles mais prises dans des sens opposés forment entre elles un angle de 180°. Dans l'exemple illustré, la direction principale d'incidence **I** et la direction principale retour **R** sont perpendiculaires, ce qui permet de limiter les risques de parasitage de la réponse lumineuse par le rayonnement incident. En variante, la direction principale d'incidence **I,** prise dans le sens allant de la source **58** vers la zone d'analyse **26',** et la direction principale retour **R,** prise dans le sens allant de la zone d'analyse **26'** vers le spectromètre **64,** pourraient être parallèles ou quasiment parallèles, mais de sens opposés, avec la source **58** et le spectromètre agencés du même côté de la zone d'analyse **26'.** De préférence, le réceptacle arrière **26** présente une fenêtre de sortie de la réponse optique qui s'étend sensiblement perpendiculairement à la direction principale retour **R.**

Dans le cas d'une analyse par transmission, notamment pour une analyse colorimétrique par la détection d'un agent optique de type chromatique, la direction principale d'incidence **I** et la direction principale retour **R** sont de préférence alignées à l'entrée et à la sortie de la zone d'analyse **26',** avec ainsi la direction principale d'incidence **I,** prise dans le sens allant de la source **58** vers la zone d'analyse **26',** et la direction principale retour **R,** prise dans le sens allant de la zone d'analyse **26'** vers le spectromètre **64** qui seraient parallèles et de même sens, sauf utilisation d'un réflecteur pour défléchir l'un des deux faisceaux.

Avantageusement, l'élément de dispersion **68** et le capteur photoélectrique **70** sont agencés à l'intérieur d'un boîtier **76** dans lequel est formé le diaphragme d'entrée **66.**

Bien entendu, pour des raisons de compacité du dispositif, on cherchera à utiliser un spectromètre **64** de faibles dimensions. Différentes versions de spectromètres utilisables dans le cadre de l'invention sont proposées par exemple par la société japonaise HAMAMATSU PHOTONICS KK, 325-6, Sunayama-cho, Naka-ku, Hamamatsu City, Shizuoka Pref., 430-8587, Japan. Par exemple il est possible d'utiliser, dans le cadre de l'invention, un des spectromètres de la gamme « Mini-Spectrometer », telle la référence C10988MA-01, ou de la gamme « Micro-Spectrometer », telle la référence C12666MA, ou la référence C12880MA.

Dans l'exemple illustré, le boîtier **76** est un boîtier parallélépipédique qui comporte une paroi avant **78** tournée en direction de la seconde fenêtre **46** et orientée sensiblement perpendiculairement à une direction principale d'entrée du rayonnement dans le spectromètre. En effet, la paroi avant **78** est munie, sensiblement en son centre, du diaphragme d'entrée **66** ici réalisé sous la forme d'une fente. À l'intérieur du boîtier **76,** la surface de réflexion concave portant le réseau de diffraction **68** renvoie un faisceau incident, sous la forme d'une dispersion chromatique, en direction du capteur photoélectrique **70** qui est agencé contre la face interne de la paroi avant **78.** Le capteur photoélectrique **70** est ainsi décalé latéralement par rapport à la fente d'entrée **66** et sa surface sensible est tournée vers l'arrière du boîtier **76,** en regard de la surface de réflexion portant le réseau de diffraction **68.** Une telle construction a l'avantage d'une très grande compacité permettant à un tel spectromètre d'être contenu dans un boîtier **76** dont le volume peut être inférieur à 10 cm3.

Le spectromètre **64** délivre un signal électronique représentatif de la décomposition spectrale chromatique du rayonnement entrant au travers du diaphragme d'entrée **66.** Ce signal électronique est produit par un circuit électronique associé au capteur photoélectrique **70.** Le circuit électronique peut être intégré au boîtier **76,** accolé à celui-ci, ou être au moins en partie porté par la carte électronique **48** liée au chariot mobile **34.** Ce signal électronique peut comprendre un signal analogique, mais il comporte de préférence un signal numérique moins sensible aux perturbations. Ce signal peut être entièrement numérique. Il est avantageusement transmis à l'unité centrale de commande de la machine par le faisceau électrique **50.**

De préférence, le spectromètre est capable de délivrer une décomposition spectrale chromatique de la réponse optique sur au moins une plage utile de longueurs d'onde s'étendant entre une première longueur d'onde et une seconde longueur d'onde double de la première. Par exemple, une plage utile de longueurs d'onde peut s'étendre entre deux

longueurs d'onde $\lambda_{min}$ et $\lambda_{max}$, avec $\lambda_{max} \geq 2 \times \lambda_{min}$. En effet, l'utilisation d'un spectromètre embarqué sur le chariot mobile **34** permet d'obtenir des informations sur la composition du spectre de la réponse optique. L'analyse de ces informations sur une certaine plage, significative, permet, comme on le verra plus tard, d'affiner les résultats de l'analyse. Bien entendu, la plage utile du spectromètre est choisie pour correspondre aux propriétés optiques particulières de l'agent optique que l'on souhaite détecter dans la solution finale de réaction. Avantageusement, la plage utile du spectromètre **64** sera telle qu'elle pourra couvrir deux propriétés optiques prédéterminées distinctes, notamment deux longueurs d'onde distinctes, correspondant chacune à au moins 2 agents optiques distincts, voire plus de 2 agents optiques distincts.

On comprend qu'une machine telle que décrite ci-dessus est avantageusement utilisée lorsque la solution de réaction comporte au moins un agent fluorescent dont la présence et/ou la quantité dans la solution de réaction dépend de la présence et/ou de la quantité d'un analyte recherché dans l'échantillon. Bien entendu, on veille alors à ce que le spectre d'émission de l'agent fluorescent est au moins partiellement compris dans la plage utile de longueurs d'onde du spectromètre.

De préférence, le rayonnement électromagnétique d'excitation émis par la source de rayonnement **58** présente un spectre qui est distinct du spectre d'émission de l'agent optique recherché. Encore plus préférentiellement, les deux spectres sont disjoints au sens que chacun d'eux est contenu dans une plage de longueurs d'ondes et que les deux plages de longueur d'ondes ne se chevauchent pas. Cela permet notamment de limiter encore le risque de parasitage de la réponse lumineuse par le rayonnement d'excitation.

Le dispositif de lecture optique peut par ailleurs comporter des moyens de détection de l'intensité du rayonnement émis par la source de rayonnement.

Ces moyens peuvent comporter un circuit ou algorithme d'estimation de l'intensité émise par la source **58** en fonction par exemple des paramètres de contrôle de la source **58** et de caractéristiques connues de la source.

Cependant ces moyens peuvent comprendre un détecteur photoélectrique mesurant l'intensité effective du rayonnement d'excitation émis par la source **58**.

Dans l'exemple de réalisation illustré sur la **figure 4,** les moyens de détection de l'intensité du rayonnement émis par la source de rayonnement d'excitation **58** incluent un capteur photoélectrique secondaire **80,** distinct du spectromètre **64.** Le capteur photoélectrique secondaire **80** est par exemple une photodiode. Le capteur photoélectrique secondaire **80** est sensible à la longueur d'onde ou pour la plage de longueurs d'onde émise par la source **58.**

Avantageusement, le dispositif de lecture optique comporte un système de guidage du rayonnement d'excitation qui guide une partie du rayonnement incident vers le capteur photoélectrique secondaire, sans passer par la zone d'analyse. Dans l'exemple de réalisation de la **figure 4,** ce système de guidage comporte par exemple le séparateur de faisceau, par exemple sous la forme d'une lame semi-réfléchissante à faces parallèles **63,** qui dirige une partie du rayonnement émis par la source **58** vers le capteur secondaire **80.** Cette partie du rayonnement émis, qui est séparée par le séparateur de faisceau **63,** sera désignée comme étant le faisceau de référence. Le système de guidage peut comporter un filtre **82** et/ou par exemple une ou plusieurs lentilles, voire un ou plusieurs éléments parmi des fibres optiques, des prismes, des miroirs et/ou un guide optique de forme tronconique, tel qu'illustré à la **figure 4** en aval de filtre **82,** pour acheminer le faisceau de référence vers le capteur secondaire **80.** Le faisceau de référence ne traverse pas la zone d'analyse **26'** dans son trajet entre la source **58** et le capteur secondaire **80.** De la sorte, le capteur secondaire **80** est capable de délivrer une information, par exemple sous la forme d'un signal électrique de référence, qui est une image de l'intensité du rayonnement émis par la source **58.**

On a illustré sur la **figure 5** un deuxième mode de réalisation d'un dispositif de lecture utilisé dans l'invention, qui est identique en tout point au premier mode de réalisation illustré à la **figure 4,** sauf en ce qui concerne les moyens de détection de l'intensité du rayonnement émis par la source **58,** et la présence éventuelle d'un filtre **72** sur la trajectoire de la réponse lumineuse.. Contrairement au mode de réalisation de la **figure 4,** ces moyens de détection de l'intensité du rayonnement émis par la source **58** ne font pas appel à un capteur secondaire distinct du spectromètre **64** mais font appel au spectromètre **64** pour détecter l'intensité du rayonnement émis par la source **58.** De manière similaire au mode de réalisation de la **figure 4,** les moyens de détection comportent un système de guidage du faisceau de référence qui est prélevé par le séparateur de faisceau **63.** Le système de guidage peut comprendre des éléments identiques ou similaires à ceux qui ont été évoqués pour le système de guidage dans le mode de réalisation précédent. En revanche, le système de guidage conduit le faisceau de référence au diaphragme d'entrée **66** du spectromètre **64.** Ce mode de réalisation est avantageux en ce qu'il ne nécessite pas de composant optoélectronique distinct pour la détection de l'intensité du signal électromagnétique émis par la source **58.** On notera que le système de guidage du faisceau de référence, dans ce second mode de réalisation, assure lui aussi que le faisceau de référence ne passe pas par la zone d'analyse **26'.** De plus, dans l'exemple illustré, le faisceau de référence ne passe pas par un filtre après sa réflexion par le séparateur de faisceaux **63.**

Ce second mode de réalisation est particulièrement avantageux dans le cas où le spectre d'émission de la source **58** est distinct, préférentiellement disjoint, de la propriété optique caractéristique de l'agent optique que l'on souhaite détecter. En effet, dans ce cas, l'information de décomposition spectrale fournie par le spectromètre **64** permettra d'identifier très clairement et distinctement le ou les pics d'intensité correspondant à des longueurs d'onde distinctes pour, d'un côté, le rayonnement émis par la source **58** et, d'un autre côté, le rayonnement correspondant à la réponse optique de la solution finale de réaction analysée.

On notera que, dans ce second mode de réalisation, le faisceau de référence est mélangé à la réponse optique en provenance de la zone d'analyse avant l'entrée au travers du diaphragme **66** du spectromètre **64.**

Toutefois, en variante non conforme à l'invention, de ce second mode de réalisation, on pourrait aussi prévoir d'acheminer alternativement uniquement l'un ou l'autre du faisceau de référence ou de la réponse lumineuse vers le spectromètre **64,** en occultant l'autre. Dans ce cas, le spectromètre **64** détermine successivement l'intensité du faisceau de référence puis la décomposition spectrale chromatique de la réponse optique de la zone d'analyse **26'.** Cette variante, qui conserve l'avantage d'un capteur photoélectrique unique, peut être utile en cas de chevauchement au moins partiel du spectre du rayonnement d'excitation émis par la source **58** avec le spectre de la réponse optique de la zone d'analyse **26'.**

On notera que, dans le mode de réalisation de la **figure 5,** on peut prévoir, à titre optionnel, un filtre optique **72** sur la trajectoire de la réponse lumineuse. De préférence, un tel filtre laisse passer les longueurs d'onde de la plage utile du spectromètre, nécessaires à la détection des agents optiques recherchés. L'absence d'un filtre sur la trajectoire de la réponse lumineuse permettrait cependant de faire fonctionner le système avec des agents optiques différents qui émettent des longueurs d'ondes différentes, sans nécessiter d'adaptation physique. De même, la présence d'un filtre sur la trajectoire du faisceau de référence est possible, mais optionnelle. L'absence d'un tel filtre, comme illustré sur la **figure 5,** permet de faire fonctionner le système avec des sources **58** de longueurs d'ondes différentes, sans nécessiter d'adaptation physique du système.

Dans la suite, on décrira un procédé d'analyse, tel que permis par l'utilisation d'une machine selon l'invention, dans le cadre d'une application dans laquelle l'agent optique fluorescent émet un rayonnement ayant une longueur d'onde définie ou une plage étroite de longueurs d'onde définie ou ayant une décomposition spectrale présentant un ou plusieurs pics caractéristiques dans une plage de longueurs d'onde définie. Bien entendu, le spectromètre **64** possède une plage utile couvrant cette longueur d'onde ou cette plage de longueurs d'onde correspondant à l'agent fluorescent. Avantageusement, l'agent fluorescent est choisi pour que sa fluorescence soit déclenchée par l'excitation d'un rayonnement dont la longueur d'onde est différente de la longueur d'onde de fluorescence ou de la plage de longueurs d'onde de fluorescence. Par exemple, l'invention peut être utilisée dans le cadre d'une application mettant en œuvre un agent fluorescent communément appelé 4-MU (4-méthylumbelliférone) qui est susceptible d'être excité par un rayonnement dans le domaine ultraviolet, par exemple par un rayonnement ayant une longueur d'onde de 370 nm (+/- 5 nm), et qui émet un rayonnement de fluorescence dans une plage de longueurs d'onde dont le pic est situé à environ 450 nm (mais dont la plage de rayonnement peut s'étendre de 375 à 550 nm environ). Un autre exemple d'agent fluorescent est le « Quanta-Blu® », disponible auprès de Thermo Fisher Scientific, Pierce Biotechnology, P.O. Box 117, Rockford, IL 61105 United States, qui présente un pic d'émission de fluorescence à 420 nm lorsque excité avec un rayonnement d'excitation à 325 nm. Du même fournisseur, on peut utiliser par exemple les agents Qdot® 605, Qdot® 705, ou Qdot® 525. A chaque fois, il faut cependant prendre soin de veiller à ce que la source lumineuse soit adaptée pour qu'elle émette bien un rayonnement d'excitation approprié.

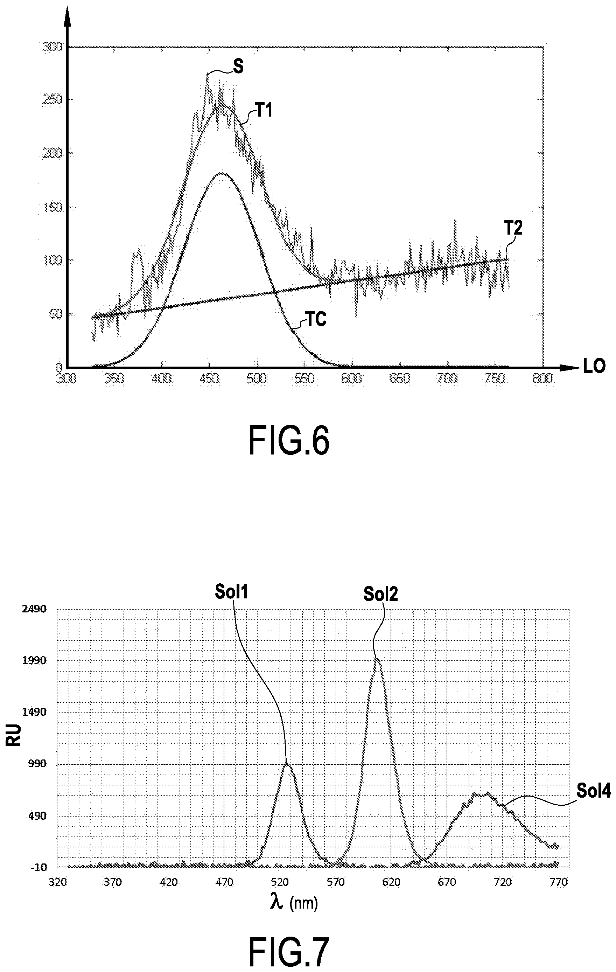

Sur la **figure 6,** on a illustré la décomposition spectrale chromatique délivrée par le spectromètre **64** dans le cas où a été placée, dans une zone d'analyse **26',** une solution contenant un agent fluorescent 4-MU, cette zone d'analyse étant illuminée par la source de rayonnement d'excitation **58** constituée dans le cas présent d'une diode électroluminescente dans le domaine ultraviolet émettant notamment dans une plage de longueurs d'onde étroite de 370 nm +/- 5 nm. Le graphe de la **figure 6** donne, en ordonnée, une grandeur image d'une intensité d'une composante chromatique du rayonnement, en fonction, selon l'axe des abscisses, de la longueur d'onde **LO** de la composante chromatique du rayonnement reçu par le spectromètre **64.**

Le signal délivré par le spectromètre **64** est un signal **S** irrégulier. Cependant, ce signal présente une forme générale qui de toute évidence présente un pic d'intensité dont le maximum est atteint aux alentours de 450 nm de longueur d'onde. Toutefois, on remarque que, de part et d'autre du pic d'intensité qui s'étend approximativement de 350 à 550 nm, le signal **S** n'est pas nul. Cette partie non nulle du signal témoigne de perturbations ou d'éléments parasites qui ne sont pas directement liés à la fluorescence de l'agent fluorescent.

Un deuxième aspect de l'invention consiste donc à proposer un procédé permettant de mieux isoler la caractéristique intéressante du signal délivré par le spectromètre, caractéristique qui correspond effectivement à la propriété optique caractéristique de l'agent optique que l'on veut détecter et/ou quantifier.

Aussi, selon un aspect de l'invention, il est proposé un procédé permettant d'affiner le résultat obtenu par une analyse.

Un tel procédé pourra être utilisé pour la détection et/ou la quantification automatisée in vitro d'analytes contenus dans des échantillons. Il pourra avantageusement être mis en œuvre à l'aide d'une machine selon l'invention.

Dans un tel procédé, on peut mettre l'échantillon en situation de réagir avec un ou plusieurs réactifs pour produire directement ou indirectement une solution de réaction comportant, en présence d'un analyte déterminé dans l'échantillon, un agent ayant au moins une propriété optique prédéterminée, par exemple une couleur, une phosphorescence, une luminescence. De préférence, l'agent optique n'est présent dans la solution de réaction ou ne possède la propriété optique prédéterminée qu'en présence de l'analyte déterminé. De préférence, l'intensité de la propriété optique prédéterminée est une fonction de la quantité ou concentration de l'analyte dans l'échantillon déterminée. Avantageusement, cette propriété optique prédéterminée est détectable par analyse d'une décomposition spectrale chromatique d'un rayonnement émis par la solution de réaction lorsque ce rayonnement est soumis à un rayonnement électromagnétique incident. L'agent optique est par exemple un agent fluorescent et la propriété optique prédéterminée est alors l'émission fluorescente, qui peut par exemple être caractérisée par une décomposition spectrale chromatique particulière.

Cette étape peut être mise en œuvre dans la machine telle que décrite ci-dessus ou peut-être réalisée dans une autre machine ou manuellement ou par tout autre moyen.

Ensuite, le procédé prévoit d'illuminer la solution de réaction avec un rayonnement électromagnétique d'excitation. Pour ce faire, la solution de réaction est disposée dans une zone d'analyse, coïncidant par exemple avec le réceptacle arrière **26** d'une barrette d'analyse **22** telle que décrite ci-dessus. L'illumination peut être réalisée par la source **58** de la machine décrite ci-dessus.

Le procédé prévoit de détecter la réponse optique de la solution de réaction et de déduire de la réponse optique la présence et/ou la quantification de l'analyte. Avantageusement, la détection de la réponse optique se fait grâce à la machine décrite ci-dessus et notamment par interprétation de la décomposition spectrale chromatique de la réponse optique qui est obtenue grâce au spectromètre **64.**

Selon une étape du procédé, on détermine une relation théorique approximée de variation de l'intensité en fonction de la longueur d'onde, image de la décomposition spectrale chromatique de la réponse optique.

Cette relation théorique approximée peut être obtenue de manière graphique ou de manière mathématique. Elle est obtenue avantageusement par toute méthode connue d'ajustement de courbe. Ces méthodes d'ajustement de courbes peuvent comprendre un ajustement par la méthode des moindres carrés.

Sur la **figure 6,** on a représenté un exemple dans lequel on a identifié deux parties de cette relation théorique. Une partie **T1** qui correspond au mieux au pic d'intensité s'étendant entre 350 et 550 nanomètres environ. Cette partie **T1** peut être prédéfinie comme étant une courbe polynomiale ou une courbe de Gauss ou tout autre courbe paramétrique, et il est alors possible, par des méthodes de régression connues, de déterminer la partie **T1** de la relation théorique. Une partie **T2** de la relation théorique est une relation linéaire qui correspond au mieux au signal **S** en dehors du pic d'intensité s'étendant entre 350 et 550 nanomètres. Cette partie **T2** de la relation théorique peut être déterminée par exemple par régression linéaire.

Ainsi, on peut déterminer que, dans la relation théorique approximée de variation de l'intensité en fonction de la longueur d'onde, un décalage linéaire de l'intensité, fonction linéaire de la longueur d'onde, affecte l'ensemble de la courbe. Dans l'exemple illustré, ce décalage linéaire peut être assimilé à la partie linéaire **T2** de la relation théorique. Dans la réalité, ce décalage linéaire peut être lié par exemple au moins en partie à une absorption différentielle du rayonnement par le matériau constituant le réceptacle arrière **26** de la barrette d'analyse **22.**

Sur cette base, on peut alors déterminer une relation théorique corrigée de la variation de l'intensité en fonction de la longueur d'onde, en corrigeant la relation théorique approximée en fonction du décalage linéaire. Par exemple, on peut tout simplement soustraire point par point le décalage linéaire de la relation théorique approximée. Par cette soustraction, on aboutit ainsi à une courbe corrigée **TC,** que l'on peut assimiler à la partie de la réponse optique purement liée à la propriété caractéristique de l'agent optique, par exemple sa propriété de fluorescence.

De la courbe corrigée **TC** on peut déduire un pic d'intensité correspondant à l'intensité maximale ou correspondant à la valeur moyenne d'intensité sur la largeur du pic.

On comprend donc que la décomposition spectrale chromatique obtenue dans ce procédé, ce qui est rendu possible par l'utilisation d'un spectromètre dans la machine selon l'invention, permet d'utiliser des valeurs de mesure de la réponse optique, en dehors du domaine de longueurs d'onde de la propriété caractéristique de l'agent optique, pour affiner la détection dans le domaine de longueurs d'onde de la propriété caractéristique de l'agent optique.

Par ailleurs, il a été vu que, dans l'art antérieur, les machines connues mettent en œuvre des procédés dans lesquels, au sein d'une même barrette d'analyse, on ne peut conduire qu'une seule analyse du fait de la détection d'un seul agent optique dans une solution finale de réaction donnée contenue dans une zone d'analyse donnée.

Selon l'invention, il est proposé un nouveau procédé permettant, dans le cadre d'une même analyse, d'obtenir plus d'informations. Ce procédé peut être mis en œuvre en utilisant une machine selon l'invention telle que décrite plus haut, utilisant un spectromètre.

De même que le procédé décrit plus haut, ce procédé peut être mis en œuvre, notamment à l'aide d'une machine selon l'invention, pour la détection et/ou la quantification automatisée in vitro d'analytes contenus dans des échantillons. De manière similaire à ce qui a été décrit plus haut, ce procédé comporte les étapes suivantes :

- mettre l'échantillon en situation de réagir avec un ou plusieurs réactifs pour produire directement ou indirectement une solution de réaction comportant, en présence d'un analyte déterminé dans l'échantillon, un agent optique ayant au moins une propriété optique prédéterminée;
- illuminer la solution de réaction avec un rayonnement électromagnétique ;
- détecter la réponse optique de la solution de réaction ;

- déduire de la réponse optique la présence et/ou la quantification de l'analyte.

Dans le détail, ces différentes étapes peuvent être identiques ou similaires aux étapes correspondantes décrites dans le cadre du procédé précédent. Ces détails ne sont donc pas répétés ici.

Ce nouveau procédé met en œuvre un ou plusieurs réactifs pour produire directement ou indirectement une solution de réaction comportant au moins deux agents optiques distincts ayant chacun au moins une propriété optique prédéterminée (couleur, fluorescence, phosphorescence, ..., telle que définie plus haut), les deux propriétés optiques prédéterminées, correspondant chacune à un agent optique, étant distinctes. Ainsi, les deux agents optiques peuvent présenter une couleur différente, une fluorescence différente, une phosphorescence différente, ... toutes propriétés qui peuvent être distinguées par leur décomposition spectrale chromatique.

Le procédé comporte les étapes consistant à acquérir une décomposition spectrale chromatique de la réponse optique, et à détecter séparément les deux propriétés optiques prédéterminées distinctes dans ladite décomposition spectrale.

De préférence, le procédé prévoit d'acquérir une décomposition spectrale unique, sous un rayonnement électromagnétique incident donné. Dans ce cas, les propriétés optiques seront détectées séparément au sens qu'elles pourront être chacune identifiées dans une même décomposition spectrale. Par exemple, chaque propriété optique pourra se traduire par la présence d'un pic d'intensité ayant un maximum pour une longueur d'onde différente. Dans ce cas, les pics d'intensité correspondront de préférence à des plages de longueurs d'ondes distinctes, plus préférentiellement disjointes.

En variante, le procédé prévoit d'acquérir plusieurs décompositions spectrales chromatiques, acquises en illuminant la zone d'analyse avec des rayonnements électromagnétiques incidents différents (notamment différents par leur décomposition spectrale chromatique). Dans ce cas, les propriétés optiques prédéterminées distinctes seront détectées séparément au sens qu'elles pourront être chacune identifiées dans des décompositions spectrales chromatiques distinctes.

L'étape de détection d'une propriété optique prédéterminée peut être réalisée en détectant l'absence de cette propriété optique prédéterminée.

Ce procédé peut être mis en œuvre pour détecter la présence d'au moins deux analytes distincts dans l'échantillon. Dans ce cas, les différents agents optiques correspondent à des analytes distincts. Il peut aussi être mis en œuvre pour détecter au moins deux réactions distinctes mettant en œuvre un même analyte. Dans ce cas, les différents agents optiques correspondent à des réactions distinctes pour un même analyte.

Dans tous les procédés décrits ci-dessus, l'étape consistant à acquérir une décomposition spectrale chromatique de la réponse optique comporte une étape de dispersion chromatique d'un faisceau optique issu de la réponse optique et une étape de détection de l'intensité par composantes de longueurs d'onde distinctes du faisceau ainsi dispersé.

De même, chacun des procédés ci-dessus peut comporter une étape de détection de l'intensité du rayonnement émis par la source de rayonnement.

Cette étape peut comporter une étape de guidage d'une partie du rayonnement incident, sans passer par la zone d'analyse, et une étape consistant à acquérir une décomposition spectrale chromatique de ladite partie du rayonnement incident formant un faisceau de référence, notamment grâce à un spectromètre, tel que mis en œuvre dans le dispositif illustré à la **figure 5.** Dans ce cas, avant l'étape consistant à acquérir une décomposition spectrale chromatique du rayonnement incident, on peut prévoir une étape de mélange de ladite partie du rayonnement incident et de la réponse optique de la solution de réaction, par exemple comme mis en œuvre dans le dispositif illustré à la **figure 5.**

Par ailleurs, l'invention peut être perfectionnée en utilisant un procédé et un système tel que décrit dans le document WO2013/045807 A1. Dans ce document, il est décrit un système et un procédé de modulation de la source et de démodulation des signaux de détection et de référence, dans le but d'améliorer le rapport signal / bruit de la mesure de détection. Le système et le procédé sont décrits dans le document WO2013/045807 A1 dans le cadre où il n'y a qu'un seul signal de détection. Le même principe de modulation et de détection peut être appliqué dans le cadre de l'invention. On peut ainsi, moduler la source de rayonnement **58** en amplitude à une fréquence / longueur d'onde porteuse. Ensuite, on peut configurer le signal de sortie du spectromètre, analogique ou numérique, qui couvre une plage utile de longueurs d'onde, en une série de N signaux élémentaires qui sont chacun représentatifs de l'intensité du signal recueilli par le spectromètre pour une longueur d'onde élémentaire ou plage élémentaire de longueurs d'onde. De préférence, les N longueurs d'onde élémentaires ou gammes élémentaires de longueurs d'onde couvrent la plage utile de longueurs d'onde, de préférence de manière continue ou quasi-continue. Pour chaque signal élémentaire, ou au moins pour une série, de préférence représentative, de ces signaux élémentaires, on peut individuellement mettre en œuvre le système et le procédé de modulation et de démodulation décrits dans le document WO2013/045807 A1. En appliquant cet enseignement à l'ensemble des signaux élémentaires, ou à une série de ces signaux, le résultat est une décomposition spectrale chromatique de la réponse optique qui présente un meilleur rapport signal / bruit, c'est-à-dire une décomposition moins perturbée par le bruit ambiant à la mesure.

L'invention n'est pas limitée aux exemples décrits et représentés car diverses modifications peuvent y être apportées sans sortir de son cadre qui est défini par les revendications.

Partie Expérimentale

Ce qui suit présente une expérience montrant la capacité d'une machine et d'un procédé selon l'invention d'acquérir, en une seule acquisition, la réponse lumineuse correspondant à trois agents optiques, avec la capacité de quantifier la présence de chaque agent optique.

Dans cette expérience, trois agents optiques ont été utilisés commercialisés par Thermo Fisher, 3747 N. Meridian Road, PO Box 117, Rockford, IL 61105 USA :

- Agent A : F(ab')2-Goat anti-Mouse IgG (H+L) Secondary Antibody, Qdot® 605 conjugate;
- Agent B : F(ab')2-Goat anti-Mouse IgG (H+L) Secondary Antibody, Qdot® 705 conjugate;
- Agent C : F(ab')2-Goat anti-Mouse IgG (H+L) Secondary Antibody, Qdot® 525 conjugate

La source lumineuse utilisée est une source monochromatique émettant dans l'ultra-violet (370 nm +/- 5nm) pour exciter les molécules

Des solutions des agents A, B, et C ont été préparées en utilisant un agent tampon CHES (acide N-Cyclohexyl-2-aminoethanesulfonic), avec un pH de 9.2.

5 Solutions ont été préparées :

| Sol 1 | Agent C 200 nM |
|-------|----------------|
| Sol 2 | Agent A 20 nM |
| Sol 3 | Agent A 200 nM |
| Sol 4 | Agent B 20nM |
| Sol 5 | Agent A 20nM<br>Agent B 20nM<br>Agent C 200nM |

Un volume de 250 microlitres de chaque solution a été placé dans une zone d'analyse et la réponse lumineuse de chaque solution a été acquise grâce à une machine selon l'invention.

Sur la **figure 7** on a superposé les décompositions spectrales des réponses lumineuses obtenues séparément pour les solutions 1, 2 et 4. Chaque décomposition spectrale présente donc un pic bien identifié. Les courbes sont exprimées en unités d'intensité relative RU de la réponse lumineuse, en fonction de la longueur d'onde $\lambda$ en nm.

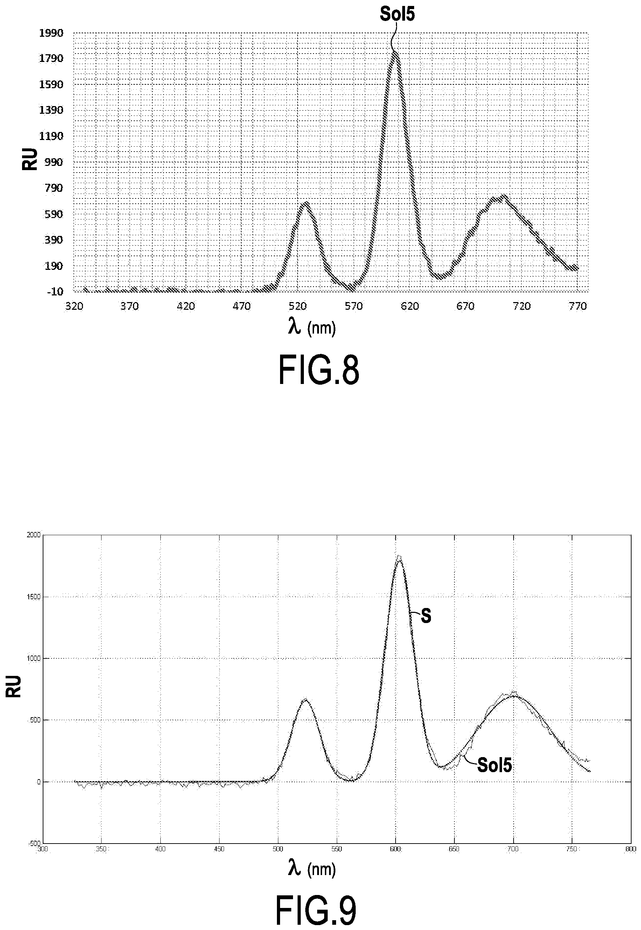

Sur la **figure 8,** on a illustré la décomposition spectrale de la réponse lumineuse obtenue séparément pour la solution 5, comprenant un mélange des trois agents. On y retrouve les trois pics, donc on voit la possibilité de détecter séparément, dans une même analyse, trois agents optiques distincts. La courbe est exprimée en unités d'intensité relative RU de la réponse lumineuse, en fonction de la longueur d'onde $\lambda$ en nm.

En utilisant les données de la décomposition spectrale illustrée à la **figure 8,** on a appliqué un algorithme de régression gaussienne dynamique pour discriminer les réponses dues aux trois agents optiques.

Ainsi, le signal de la **figure 8** a été modélisé par la somme de trois fonctions gaussiennes appelées respectivement G525, G605 et G705, centrées chacune sur le pic de fluorescence de l'agent optique correspondant avec:

$$G525(X, A_1, \sigma_1, \mu_1) = A_1 \times \frac{1}{\sqrt{2\pi\sigma_1}} \times e^{-\frac{(X-\mu_1)^2}{2\sigma_1}}$$

$$G605(X, A_2, \sigma_2, \mu_2) = A_2 \times \frac{1}{\sqrt{2\pi\sigma_2}} \times e^{-\frac{(X-\mu_2)^2}{2\sigma_2}}$$

$$G705(X, A_3, \sigma_3, \mu_3) = A_3 \times \frac{1}{\sqrt{2\pi\sigma_3}} \times e^{-\frac{(X_3-\mu_3)^2}{2\sigma_3}}$$

où :

- X est la Variable, qui correspond ici à la longueur d'onde
- A, $\sigma$ et $\mu$ sont des paramètres relatifs à l'amplitude, l' écart-type et la valeur moyenne de la chaque fonction gaussienne.

Ainsi, le modèle utilisé pour modéliser la courbe de la **figure 8,** illustrant la décomposition spectrale de la réponse lumineuse obtenue séparément pour la solution 5 peut s'écrire :

$$\boldsymbol{S}(X, A_1, \sigma_1, \mu_1, A_2, \sigma_2, \mu_2, A_2, \sigma_3, \mu_3)$$
$$= \boldsymbol{G525}(A_1, \sigma_1, \mu_1) + \boldsymbol{G605}(A_2, \sigma_2, \mu_2) + \boldsymbol{G705}(A_3, \sigma_3, \mu_3)$$

Le vecteur de paramètres à résoudre par optimisation comprend donc neuf paramètres :

$$P = (A_1, \sigma_1, \mu_1, A_2, \sigma_2, \mu_2, A_3, \sigma_3, \mu_3)$$

Le processus d'optimisation utilisé est celui disponible par le logiciel Matlab et utilisant l'algorithme appelé « Flexible Simplex Method ». Cet algorithme minimise la fonction de coût qui est la somme quadratique des erreurs, représentant l'erreur entre le modèle (avec les valeurs instantanées des paramètres du vecteur pour l'itération considérée) et la courbe expérimentale. A chaque itération, le vecteur P est modifié selon l'algorithme et la somme quadratique des erreurs est vérifiée. Les itérations sont arrêtées lorsqu'on on atteint une erreur inférieure à un seuil donné qui dépend de la précision souhaitée.

Les valeurs initiales de paramètres, utilisées pour le processus d'optimisation, sont :

| $A1_0$ | $\sigma1_0$ | $\mu1_0$ | $A2_0$ | $\sigma2_0$ | $\mu2_0$ | $A3_0$ | $\sigma3_0$ | $\mu3_0$ |
|------|------|-------|------|------|-------|------|------|-------|
| 1000 | 525 | 21270 | 170 | 605 | 30270 | 1000 | 705 | 15270 |

Ces valeurs permettent d'aboutir rapidement à la solution, mais d'autres valeurs initiales pourraient être utilisées.

Le résultat de l'optimisation a donné les paramètres suivants (arrondis à l'unité inférieure):

| $\sigma1$ | $\mu1$ | A1 | $\sigma2$ | $\mu2$ | A2 | $\sigma3$ | $\mu3$ | A3 |
|-----|-----|-------|-----|-----|-------|-----|-----|-------|
| 144 | 523 | 19876 | 145 | 603 | 54083 | 988 | 700 | 54574 |

La **figure 9** illustre la courbe de la fonction S obtenue avec ces paramètres optimisés, en superposition avec la courbe de la **figure 8.** Les courbes sont exprimées en unités d'intensité relative RU de la réponse lumineuse, en fonction de la longueur d'onde $\lambda$ en nm.

Les trois fonctions G525, G605 et G705 définies avec ces paramètres optimisés permettent d'extraire de la réponse lumineuse globale illustrée à la **figure 8** les réponses lumineuses dues individuellement à chaque agent optique. En se référant à une échelle de calibration adéquate, on peut aisément quantifier la concentration de chaque agent optique dans la solution unique analysée.

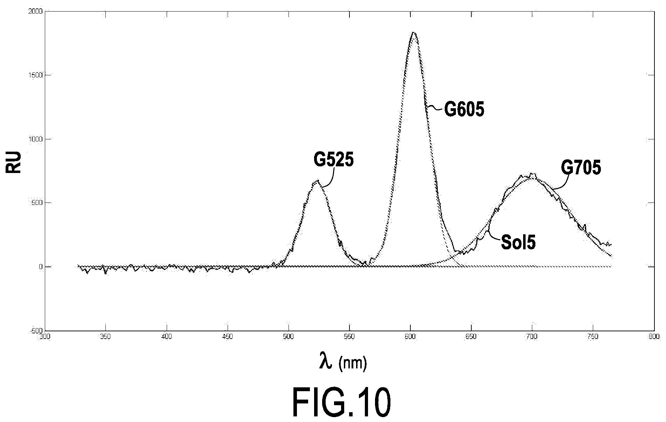

La **figure 10** illustre les trois fonctions G525, G605 et G705 obtenues avec ces paramètres superposés avec la courbe de la **figure 8.** Les courbes sont exprimées en unités d'intensité relative RU de la réponse lumineuse, en fonction de la longueur d'onde $\lambda$ en nm.

Cette expérience montre comment une machine et/ou un procédé selon l'invention peuvent être mis en œuvre pour quantifier, en une seule analyse, la présence de plusieurs agents optiques distincts qui peuvent témoigner de plusieurs réactions d'analyse.

En particulier :

- un technicien de laboratoire, ou la machine, prépare un échantillon à analyser pouvant comprendre plusieurs types d'analytes différents ;

- une sélection de plusieurs agents optiques différents, présentant des propriétés optiques distinctes, préférentiellement dans des plages optiques différentes, e.g. disjointes, sont introduits à escient dans l'échantillon. Chacun de ces agents vise un type d'analyte particulier dont il est recherché la présence ou la concentration/quantité dans l'échantillon ;

- une réponse optique est mesurée par le spectromètre pour l'échantillon, réponse qui comprend par conséquent la réponse de chaque agent optique si l'analyte correspondant est présent dans l'échantillon, comme décrit ci-avant ; - la réponse est communiquée à l'unité centrale ou un ordinateur distant, qui en réponse met en œuvre un traitement informatique pour identifier le mélange de gaussiennes correspondant aux agents optiques de la manière décrit ci-avant.

- le traitement informatique détermine alors en fonction du chaque gaussiennes du mélange la concentration et/ou la quantité d'analyte correspondant de la manière décrite ci-avant;

- le résultat du traitement est alors par exemple mémorisé ou affiché sur un écran.

**Revendications**

1. - Procédé pour la détection et/ou la quantification automatisée in vitro d'analytes contenus dans des échantillons, comportant les étapes suivantes :

   - mettre l'échantillon en situation de réagir avec un ou plusieurs réactifs pour produire directement ou indirectement une solution de réaction comportant, en présence d'un analyte déterminé dans l'échantillon, un agent optique ayant au moins une propriété optique prédéterminée ;
   - illuminer la solution de réaction contenue dans une zone d'analyse optique avec un rayonnement électromagnétique incident;
   - détecter la réponse optique de la solution de réaction ;
   - déduire de la réponse optique la présence et/ou la quantification de l'analyte ;
   le procédé mettant en œuvre un ou plusieurs réactifs pour produire directement ou indirectement une solution de réaction comportant au moins deux agents optiques distincts ayant chacun au moins une propriété optique prédéterminée, les deux propriétés optiques prédéterminées étant distinctes,
   le procédé comportant en outre les étapes consistant à:

      - acquérir une décomposition spectrale chromatique de la réponse optique et
      - détecter séparément les deux propriétés optiques prédéterminées distinctes dans ladite décomposition spectrale,

   le procédé étant **caractérisé en ce que** qu'il comporte :

      - une étape de guidage d'une partie du rayonnement électromagnétique incident, sans passer par la zone d'analyse optique,
      - une étape consistant à acquérir une décomposition spectrale chromatique de ladite partie du rayonnement incident,
      - avant l'étape consistant à acquérir une décomposition spectrale chromatique du rayonnement électromagnétique incident, une étape de mélange de ladite partie du rayonnement électromagnétique incident et de la réponse optique de la solution de réaction,
      - une étape de détection de l'intensité de la partie du rayonnement électromagnétique incident spectralement décomposée.

2. - Procédé selon la revendication 1, **caractérisé en ce que** l'étape consistant à acquérir une décomposition spectrale chromatique de la réponse optique comporte une étape de dispersion chromatique d'un faisceau optique issu de la réponse optique et de détection de l'intensité par composantes de longueurs d'onde distinctes du faisceau ainsi dispersé.

3. - Machine pour la détection et/ou la quantification automatisée in vitro d'analytes contenus dans des échantillons, comportant :

      - plusieurs zones d'analyse optique (26') destinées à recueillir chacune une solution de réaction obtenue par réaction avec l'échantillon, et

- un dispositif de lecture optique (30) capable de détecter et/ou quantifier une réponse optique de la solution de réaction à une stimulation électromagnétique, le dispositif de lecture optique comportant au moins :

(i) une source de rayonnement électromagnétique (58) capable d'illuminer une desdites zones d'analyse optique (26'), (ii) un capteur photoélectrique (70) agencé pour recueillir un rayonnement optique provenant de la solution de réaction contenue dans la zone d'analyse optique et capable de détecter la réponse optique de la solution de réaction,
(iii) un spectromètre (64) capable de délivrer une décomposition spectrale chromatique de la réponse optique, le capteur photoélectrique (70) faisant partie dudit spectromètre (64),

la machine étant **caractérisée en ce que** le dispositif de lecture (30) est porté par un chariot mobile (32) de la machine qui est motorisé et dont les déplacements sont commandés automatiquement pour amener le dispositif de lecture (30) dans différentes positions correspondant chacune à une desdites zones d'analyse (26'),
et **en ce que** le dispositif de lecture optique (30) comporte des moyens de détection de l'intensité d'un rayonnement incident émis par la source de rayonnement, et **en ce que** les moyens de détection de l'intensité du rayonnement incident émis par la source de rayonnement (58), incluent le spectromètre (64) et un système de guidage (63) configuré pour guider une partie du rayonnement incident vers le spectromètre, sans passer par la zone d'analyse optique (26') et pour mélanger ladite partie du rayonnement incident et la réponse optique de la solution de réaction.

4. - Machine selon la revendication 3, dans laquelle le spectromètre (64) est capable de délivrer une décomposition spectrale chromatique de la réponse optique sur au moins une plage utile de longueurs d'onde entre deux longueurs d'onde λmin et λmax, avec λmax> 2 x λmin.

5. - Machine selon l'une des revendications 3 ou 4, dans laquelle le spectromètre (64) comporte un élément de dispersion chromatique (68) et le capteur photoélectrique (70) est linéaire ou bidimensionnel.

6. - Machine selon l'une des revendications 3 à 5, dans laquelle le spectromètre (64) comporte un circuit électronique qui est connecté au capteur photoélectrique (70) et qui délivre un signal électrique image de la décomposition spectrale chromatique de la réponse optique.

7. - Machine selon la revendication 6, dans laquelle le signal électrique image est un signal analogique.

8. - Machine selon l'une des revendications 3 à 7, dans laquelle la source de rayonnement est une source monochromatique.

9. - Machine selon l'une des revendications 3 à 7, dans laquelle la source de rayonnement est une source polychromatique.

**Patentansprüche**

1. Verfahren zur automatisierten In-vitro-Detektion und/oder -Quantifizierung von in Proben enthaltenen Analyten, umfassend die folgenden Schritte:

- Versetzen der Probe in die Situation, mit ein oder mehreren Reagenzien zu reagieren, um direkt oder indirekt eine Reaktionslösung zu erzeugen, die, bei Vorkommen eines bestimmten Analyten in der Probe, ein optisches Mittel umfasst, das mindestens eine vorbestimmte optische Eigenschaft besitzt;
- Beleuchten der in einem optischen Analysebereich enthaltenen Reaktionslösung mit einer einfallenden elektromagnetischen Strahlung;
- Detektieren der optischen Antwort der Reaktionslösung;
- Ableiten des Vorkommens und/oder der Quantifizierung des Analyten aus der optischen Antwort;
wobei das Verfahren ein oder mehrere Reagenzien einsetzt, um direkt oder indirekt eine Reaktionslösung zu erzeugen, die mindestens zwei verschiedene optische Mittel umfasst, von denen jedes mindestens eine vorbestimmte optische Eigenschaft besitzt, wobei die beiden vorbestimmten optischen Eigenschaften verschieden sind,
wobei das Verfahren ferner die Schritte umfasst, die darin bestehen:

- eine chromatische spektrale Zerlegung der optischen Antwort zu erfassen und
- die beiden verschiedenen vorbestimmten optischen Eigenschaften in der spektralen Zerlegung getrennt zu detektieren,

wobei das Verfahren **dadurch gekennzeichnet ist, dass** es umfasst:

- einen Schritt des Führens eines Teils der einfallenden elektromagnetischen Strahlung, ohne durch den optischen Analysebereich zu verlaufen,
- einen Schritt, der darin besteht, eine chromatische spektrale Zerlegung des Teils der einfallenden Strahlung zu erfassen,
- vor dem Schritt, der darin besteht, eine chromatische spektrale Zerlegung der einfallenden elektromagnetischen Strahlung zu erfassen, einen Schritt des Mischens des Teils der einfallenden elektromagnetischen Strahlung und der optischen Antwort der Reaktionslösung,
- einen Schritt der Detektion der Intensität des spektral zerlegten Teils der einfallenden elektromagnetischen Strahlung.

2. Verfahren nach Anspruch 1, **dadurch gekennzeichnet, dass** der Schritt, der darin besteht, eine chromatische spektrale Zerlegung der optischen Antwort zu erfassen, einen Schritt der chromatischen Dispersion eines aus der optischen Antwort hervorgehenden optischen Strahls und der Detektion der Intensität durch verschiedene Wellenlängenkomponenten des so dispergierten Strahls umfasst.

3. Gerät zur automatisierten In-vitro-Detektion und/oder -Quantifizierung von in Proben enthaltenen Analyten, umfassend:

- mehrere optische Analysebereiche (26'), die dazu bestimmt sind, jeweils eine Reaktionslösung aufzunehmen, die durch Reaktion mit der Probe erhalten wird, und
- eine optische Lesevorrichtung (30), die in der Lage ist, eine optische Antwort der Reaktionslösung auf eine elektromagnetische Anregung zu detektieren und/oder zu quantifizieren, wobei die optische Lesevorrichtung mindestens eine Quelle elektromagnetischer Strahlung (58) umfasst, die in der Lage ist, einen der optischen Analysebereiche (26') zu beleuchten,
- einen fotoelektrischen Sensor (70), der dazu ausgebildet ist, eine optische Strahlung aufzunehmen, die aus der in dem optischen Analysebereich enthaltenen Reaktionslösung stammt, und in der Lage ist, die optische Antwort der Reaktionslösung zu detektieren, und
- ein Spektrometer (64), das in der Lage ist, eine chromatische spektrale Zerlegung der optischen Antwort auszugeben, wobei der fotoelektrische Sensor (70) zu dem Spektrometer (64) gehört,
wobei das Gerät **dadurch gekennzeichnet ist, dass** die Lesevorrichtung (30) von einem beweglichen Schlitten (32) des Geräts getragen wird, der motorbetrieben ist und dessen Bewegungen automatisch gesteuert werden, um die Lesevorrichtung (30) in unterschiedliche Positionen zu bringen, die jeweils einem Analysebereich (26') entsprechen,
und dadurch, dass die optische Lesevorrichtung (30) Einrichtungen zur Detektion der Intensität einer von der Strahlungsquelle ausgesendeten einfallenden Strahlung umfasst, wobei die Einrichtungen zur Detektion der von der Strahlungsquelle (58) ausgesendeten einfallenden Strahlung das Spektrometer (64) beinhalten und ein Führungssystem (63), das so konfiguriert ist, dass es einen Teil der einfallenden Strahlung zum Spektrometer leitet, ohne durch die optische Analysezone (26') zu gehen, und das so konfiguriert ist, dass es den Teil der einfallenden Strahlung und die optische Reaktion der Reaktionslösung mischt...

4. Gerät nach Anspruch 3, wobei das Spektrometer (64) in der Lage ist, eine chromatische spektrale Zerlegung der optischen Antwort über mindestens einen Nutzwellenlängenbereich zwischen zwei Wellenlängen $\lambda$min und $\lambda$max mit $\lambda$max > 2 x $\lambda$min auszugeben.

5. Gerät nach einem der Ansprüche 3 oder 4, wobei das Spektrometer (64) ein Element zur chromatischen Dispersion (68) umfasst und der fotoelektrische Sensor (70) linear oder zweidimensional ist.

6. Gerät nach einem der Ansprüche 3 bis 5, wobei das Spektrometer (64) einen elektronischen Schaltkreis umfasst, der an den fotoelektrischen Sensor (70) angeschlossen ist und der ein elektrisches Signal ausgibt, das ein Abbild der chromatischen spektralen Zerlegung der optischen Antwort ist.

7. Gerät nach Anspruch 6, wobei das elektrische Abbildsignal ein analoges Signal ist.

8. Gerät nach einem der Ansprüche 3 bis 7, wobei die Strahlungsquelle eine monochromatische Quelle ist.

9. Gerät nach einem der Ansprüche 3 bis 7, wobei die Strahlungsquelle eine polychromatische Quelle ist.

**Claims**

1. Method for automated in vitro detection and/or quantification of analytes contained in samples, comprising the following steps:

   - placing the sample in a situation to react with one or more reagents to directly or indirectly produce a reaction solution comprising, in the presence of an analyte determined in the sample, an optical agent having at least one predetermined optical property;
   - illuminating the reaction solution contained in an optical analysis zone with an incident electromagnetic radiation;
   - detecting the optical response of the reaction solution;
   - deducing from the optical response the presence and/or the quantification of the analyte;
   the method implementing one or more reagents to directly or indirectly produce a reaction solution comprising at least two distinct optical agents each having at least one predetermined optical property, the two predetermined optical properties being distinct,
   the method further comprising the steps of:

   - acquiring a chromatic spectral decomposition of the optical response and
   - separately detecting the two distinct predetermined optical properties in said spectral decomposition

   the method being **characterized in that** it comprises:

   - a step of guiding a part of the incident electromagnetic radiation, without passing through the optical analysis zone,
   - a step consisting in acquiring a chromatic spectral decomposition of said part of the incident radiation,
   - before the step consisting in acquiring a chromatic spectral decomposition of the incident electromagnetic radiation, a step of mixing said part of the incident electromagnetic radiation and the optical response of the reaction solution,
   - a step of detecting the intensity of the spectrally decomposed part of the incident electromagnetic radiation.

2. Method according to Claim 1, **characterized in that** the step consisting in acquiring a chromatic spectral decomposition of the optical response comprises a step of chromatic dispersion of an optical beam derived from the optical response and of detecting the intensity by components of distinct wavelengths of the duly dispersed beam.

3. Machine for the automated in vitro detection and/or quantification of analytes contained in samples, comprising:

   - several optical analysis zones (26') each intended to collect a reaction solution obtained by reaction with the sample and
   - an optical reading device (30) capable of detecting and/or quantifying an optical response of the reaction solution to an electromagnetic stimulation, the optical reading device comprising at least a source of electromagnetic radiation (58) capable of illuminating one of the optical analysis zones (26'), and
   - a photoelectric sensor (70) arranged to collect an optical radiation originating from the reaction solution contained in the optical analysis zone and capable of detecting the optical response of the reaction solution and
   - a spectrometer (64) capable of delivering a chromatic spectral decomposition of the optical response, the photoelectric sensor (70) forming part of said spectrometer (64),
   the machine being **characterized in that** the reading device (30) is borne by a movable carriage (32) of the machine which is motorized and the movements of which are automatically controlled to bring the reading device (30) into different positions each corresponding to an analysis zone (26')
   and **in that** the optical reading device (30) comprises means for detecting the intensity of an incident radiation emitted by the radiation source, the means for detecting the intensity of the incident radiation emitted by the radiation source (58) include the spectrometer (64) and a guiding system (63) configured to guide a part of the incident radiation to the spectrometer, without passing through the optical analysis zone (26'), and configured to mix said part of the incident radiation and the optical response of the reaction solution.

4.  Machine according to Claim 3, wherein the spectrometer (64) is capable of delivering a chromatic spectral decomposition of the optical response over at least one useful range of wavelengths between two wavelengths λmin and λmax, with λmax > 2 x λmin.

5.  Machine according to one of Claims 3 or 4, wherein the spectrometer (64) comprises a chromatic dispersion element (68) and the photoelectric sensor (70) is linear or two-dimensional.

6.  Machine according to one of Claims 3 to 5, wherein the spectrometer (64) comprises an electronic circuit which is connected to the photoelectric sensor (70) and which delivers an electrical signal that is the image of the chromatic spectral decomposition of the optical response.

7.  Machine according to Claim 6,
    wherein the image electrical signal is an analogue signal.

8.  Machine according to one of Claims 3 to 7,
    wherein the radiation source is a monochromatic source.

9.  Machine according to one of Claims 3 to 7,
    wherein the radiation source is a polychromatic source.

FIG.1

FIG.2

FIG.3

FIG.4

FIG.5

FIG.6

FIG.7

**FIG.8**

**FIG.9**

FIG.10

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2003223059 A1
- EP 0864089 B1
- EP 0871863 B1
- EP 0241268 A1

- US 5757013 A
- EP 0802413 A
- WO 2004055502 A2
- WO 2013045807 A1